# EUROPEAN PATENT APPLICATION

(11) **EP 4 825 406 A1**
(43) Date of publication of application: **21.10.2026**
(21) Application number: 25215859.7
(22) Date of filing: 14.11.2025
(51) Int. Cl.: A61M 1/36

(54) **NEEDLE DISLODGEMENT DETECTION DEVICE**

(30) Priority: 17.04.2025 JP 2025068388
(71) Applicant: Genial Light Co., Ltd., Hamamatsu-shi, Shizuoka 430-0917 (JP)
(72) Inventor: SHIMOKITA, Ryo, Hamamatsu-shi, Shizuoka 430-0917 (JP); FUJITA, Tatsuyuki, Hamamatsu-shi, Shizuoka 430-0917 (JP); SUZUKI, Hisafumi, Hamamatsu-shi, Shizuoka 430-0917 (JP); YAMAMOTO, Yoshiatsu, Hamamatsu-shi, Shizuoka 430-0917 (JP)
(74) Representative: Müller Hoffmann & Partner

(57) **Abstract**

This invention provides a needle dislodgement detection device 100 having an electrode 2 that is tightly mounted on an outer circumferential surface of a tube even if a diameter of the tube changes. In order to make it possible, the electrode 2 which is detachably mounted on the outer circumferential surface of the tube 8 connected to a puncture needle 7 is made of an elastically deformable conductive material, and the electrode 2 is mounted by being pressed against the outer circumferential surface of the tube 8 in a manner that accompanies elastic deformation.

## Description

### [Field of the art]

This invention relates to a needle dislodgement detection device.

### [Background art]

Conventionally, needle dislodgement detection devices have been conceived for hemodialysis devices to detect that an arterial side puncture needle or a venous side puncture needle becomes dislodged from the patient. According to this principle, an alternating current voltage is applied to an extracorporeal blood circuit formed with an arterial side puncture needle and a venous side puncture needle as respective ends and dislodgement of needle is detected based on a measurement signal of the current flowing at that time.

To apply the alternating current voltage to the above-mentioned extracorporeal blood circuit, for example, according to the patent document 1, electrodes are removably mounted on predetermined locations of an arterial side tube and a venous side tube constituting the extracorporeal blood circuit from the outside and an alternative current voltage is applied between these electrodes.

In accordance with this method, since the electrodes are mounted on the tube retrofitted, there is an advantage of being universally applicable to various hemodialysis devices having various different needle shapes. Furthermore, since the electrodes are detachable, there is also an advantage of lowering a manufacturing cost of this method compared with a conventional device wherein electrodes are integrally incorporated into a disposable component (for example, a puncture needle or a tube) for extracorporeal blood circuit.

On the other hand, since a gap is created between the blood and the electrode by an amount of a thickness of the tube, a current measurement signal tends to be weak. To overcome this, it is necessary to tightly attach the electrode to the tube as close as possible. For example, the patent document 1 describes a tubular electrode that tightly ataches to an outer circumferential surface of the tube.

However, with the above-mentioned configuration, it is necessary to prepare electrodes for each tube having different diameter.

### [Prior art document]

### [Patent document]

[Patent document 1] Japanese Patent Publication No. 7610857

### [Outline of the invention]

### [Problem to be solved by the invention]

Then, the present invention was conceived to solve the above-mentioned problems and aims to provide a needle dislodgement detection device having an electrode that can be attached in close contact with the outer circumferential surface of a tube, even when the tube diameter changes.

### [Means to solve the problems]

Specifically, the needle dislodgement detection device in accordance with the present invention has a following configuration.
[1] The needle dislodgement detection device is to detect dislodgement of an arterial side puncture needle or a venous side puncture needle of a hemodialysis apparatus from a patient, and comprises
   a pair of electrodes detachably mounted on an outer peripheral surface of an arterial side tube connected to the arterial side puncture needle and an outer peripheral surface of a venous side tube connected to the venous side puncture needle respectively,
   a voltage application section that applies a predetermined voltage between the electrodes,
   a current measurement section that measures a current flowing between the electrodes due to the applied voltage, and
   a needle dislodgement detection section that detects dislodgement of the arterial side puncture needle or the venous side puncture needle based on a current measurement signal output by the current measurement section, and is characterized by that
   the electrodes are made of an elastically deformable conductive material and are mounted on the outer circumferential surfaces of the tubes by being pressed against the outer peripheral surfaces in a manner that accompanies elastic deformation.
   In accordance with this configuration, even if a diameter of the tube changes, since the electrodes themselves tightly attach to the outer circumferential surface of the tube due to elastic deformation of the electrodes, it becomes possible to tightly mount the same electrode on the tube having various different diameter.
   Therefore, unlike conventional methods, there is no need to prepare the electrodes corresponding to the diameter of the tube so that the standardization of components is promoted. Furthermore, since the electrode attaches tightly to the tube, it is possible to secure the same needle dislodgement detection accuracy as that of the conventional methods.
[2] The needle dislodgement detection device described in [1], wherein
   the voltage application section applies a high-frequency voltage between the electrodes, and
   the needle dislodgement detection section detects dislodgement of the arterial side puncture needle or the venous side puncture needle based on an extracted signal obtained by extracting a predetermined frequency component from the current measurement signal.
   In accordance with this configuration, since it is possible to effectively remove various noises superimposed on the current measurement signal such as a noise generated by vibration of the blood pump of the hemodialysis apparatus or a noise generated when a person touches the tube, the needle dislodgement detection accuracy can be improved.
[3] The needle dislodgement detection device described in [1] or [2], wherein
   the electrode comprises a plurality of electrode divided bodies, wherein each of the electrode divided bodies is configured to clamp the tube from the outer circumferential surface of the tube in a state that the electrode is mounted on the tube.
   In accordance with this configuration, it is possible to easily mount the electrode on the tube even after the puncture.
[4] The needle dislodgement detection device described in [1] or [3], wherein
   the electrode divided bodies are held by a plurality of holding bodies having higher rigidity than that of the electrode divided body, and the electrodes are configured to be in the mounted state by engaging the holding bodies with each other.
   Since the electrode divided bodies elastically deform, it is difficult to stably mount the electrode divided bodies on the tube. However, since the high-rigidity holding bodies are used, it is possible to securely mount each of the electrode divided bodies on the tube by clamping the tube from its surrounding area.
[5] The needle dislodgement detection device described in [3] or [4], wherein
   the electrode comprises a pair of the electrode divided bodies, and the electrode divided bodies clamp the tube from opposite directions in the mounted state.

Since a number of the electrode divided body is the minimum, it is possible to simplify the configuration or to reduce the cost.

### [Effect of the invention]

In accordance with this invention having the above configuration, even though the diameter of the tube changes, it is possible to provide the needle dislodgement detection device that has the electrode that can be closely mounted on the outer circumferential surface of the tube.

### [Brief explanation of the drawings]

[Fig. 1] A view schematically showing a configuration of a needle dislodgement detection device in accordance with one embodiment of this invention.
[Fig. 2] An exploded perspective view showing an electrode and its attachment device of this embodiment.
[Fig. 3] A perspective view showing a state prior to mounting the electrode on a tube and a state wherein the electrode is mounted on the tube in this embodiment.
[Fig. 4] A cross-sectional view showing the state prior to mounting the electrode on the tube and the state wherein the electrode is mounted on the tube in this embodiment.
[Fig. 5] A flow chart showing a method for detecting dislodgement of a needle in this embodiment.
[Fig. 6] A view of an electrode viewed from a direction of extending the tube in another embodiment of this invention.
[Fig. 7] A view of an electrode viewed from a direction of extending the tube in a further different embodiment of this invention.
[Fig. 8] A view of an electrode and an attachment device viewed from a direction of extending a tube in a further different embodiment of this invention.
[Fig. 9] A view of an electrode and an attachment device viewed from a direction of extending a tube in a further different embodiment of this invention.

### [Best modes for embodying the invention]

One embodiment of a needle dislodgement detection device in accordance with the present invention will be explained with reference to drawings. The drawings may be schematically drawn with appropriate omissions or exaggerations for ease of understanding. Furthermore, identical components are denoted by the same reference numerals, and descriptions thereof are appropriately omitted.

The needle dislodgement detection device 100 of this embodiment detects a case when an arterial side puncture needle 7(1) or a venous side puncture needle 7(2) used in a hemodialysis apparatus 10 dislodges from a patient.

### First Explanation of hemodialysis apparatus

Before explaining the needle dislodgement detection device 100, an overview of the hemodialysis apparatus 10 is explained.

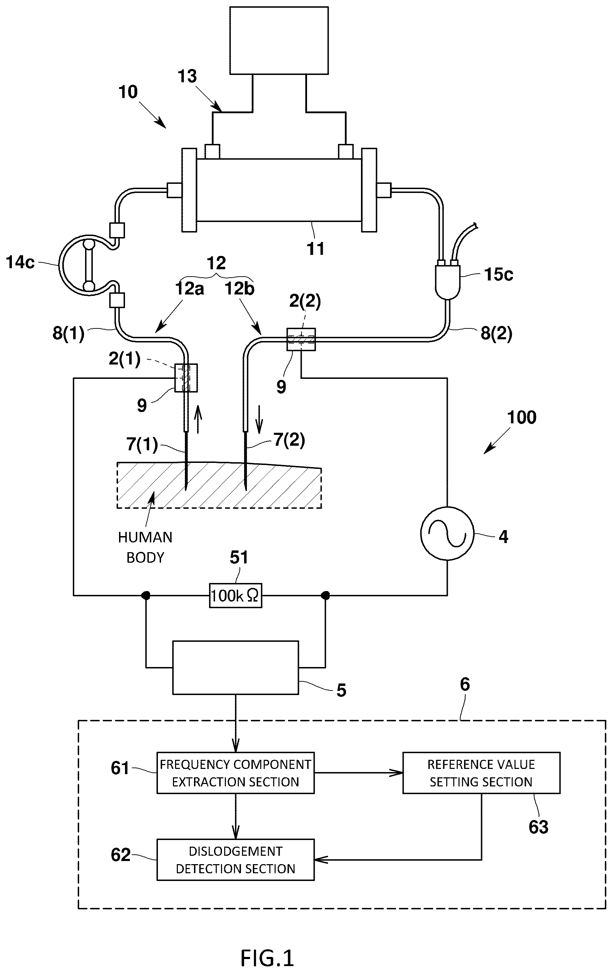

As shown in Fig. 1, the hemodialysis apparatus 10 comprises a dialyzer 11 that performs hemodialysis, an extracorporeal blood circuit 12 that is connected to the dialyzer 11 and that circulates blood, and a dialysate circuit 13 that is connected to the dialyzer 11 and that circulates dialysate. The extracorporeal blood circuit 12 has an arterial side flow path 12a and a venous side flow path 12b. The arterial side flow path 12a is formed by the arterial side puncture needle 7(1) and an arterial side tube 8(1) connected to the arterial side puncture needle 7(1) and also connected to the dialyzer 11. A reference numeral 14c in Fig. 1 denotes a blood pump provided in the arterial side tube 8(1). In addition, the venous side flow path 12b is formed by the venous side needle 7(2) and a venous side tube 8(2) connected to the venous side needle 7(2) and also connected to the dialyzer 11. A reference numeral 15c in Fig. 1 denotes a drip chamber that removes air from the blood.

### Second Explanation of needle dislodgement detection device

### 1. Overview

The needle dislodgement detection device 100, as shown in Fig. 1, comprises an arterial side electrode 2(1) detachably mounted on the arterial side tube 8(1), a venous side electrode 2(2) detachably mounted on the venous side tube 8(2), a voltage application section 4 that applies high-frequency voltage between the electrodes 2(1) and 2(2), a current measurement section 5 that detects a current flowing between the electrodes 2(1) and 2(2) to which the high-frequency voltage is applied by the voltage application section 4, and a control processing section 6 that detects dislodgement of the arterial side puncture needle 7(1) or the venous side puncture needle 7(2) based on the current measurement signal obtained by the current measurement section 5.

Hereinafter, in case that there is no need of distinguishing the arterial side and venous side for the arterial side electrode 2(1) and venous side electrode 2(2), the arterial side electrode 2(1) and venous side electrode 2(2) may be referred to simply as the electrode 2. In addition, the same applies to the arterial side tube 8(1), the venous side tube 8(2), and the arterial side puncture needle 7(1) and the venous side puncture needle 7(2).

### 2. Regarding electrode

The arterial side electrode 2(1) is detachably mounted on the arterial side tube 8(1), more concretely, on a side of the arterial side puncture needle 7(1) rather than a blood pump 14c in the arterial side tube 8(1) by making use of an attachment device 9.

The venous side electrode 2(2) is detachably mounted on the venous side tube 8(2), more specifically, on a side of the venous side puncture needle 7(2) rather than a drip chamber 15c in the venous side tube 8(2) by making use of the attachment device 9.

Moreover, the electrode 2 in this embodiment is made of a conductive elastic member, more concretely, conductive silicone rubber. The conductive elastic member is not limited to being formed from a single material like the above-mentioned conductive silicone rubber. It may also be a composite material, such as one where a surface of an elastically deformable insulating elastic member (for example, polyurethane foam, or the like) is covered with a stretchable conductive fabric.

More detail will be explained.

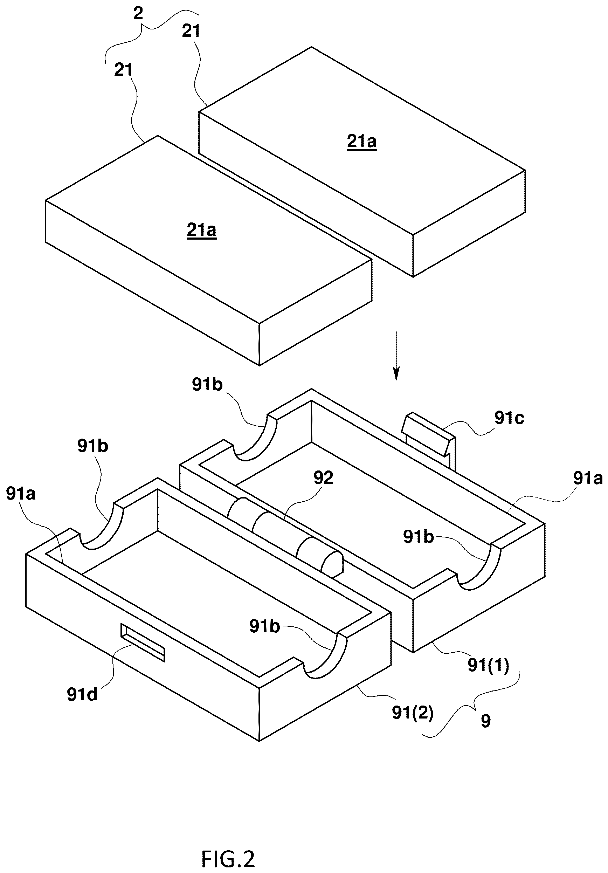

As shown in Fig. 2 to Fig. 4, the electrode 2 comprises a pair of electrode divided bodies 21. A planar portion 21a is formed on each of the opposing faces of the electrode divided bodies 21. The tube 8 is clamped and fixed between these planar portions 21a.

The attachment device 9 is made of a non-conductive resin with higher rigidity than that of the tube 8 and, as shown in Fig. 2 to Fig. 4, is formed from a pair of holding bodies 91. In the following description, in case that it is necessary to distinguish the holding bodies 91, they may be denoted as 91(1) and 91(2).

The holding body 91 is of a case shape with an opening 91a provided on one surface thereof. The electrode divided body 21 is housed and held in the holding body 91 tightly with its planar portion 21a flush with the opening 91a. In addition, notches 91b are formed at two opposing locations on a peripheral plate forming the opening 91a of the holding body 91.

An engaging portion 91c is formed on one holding body 91 (1), and an engaged portion 91d is formed on the other holding body 91 (2). Each of the holding bodies 91 is engaged with its openings 91a closely or tightly attached by engaging the engaging portion 91c with the engaged portion 91d. In this embodiment, the holding bodies 91 are connected by a hinge 92 to allow opening and closing so as not to be completely separated each other and the engaging portion 91c and the engaged portion 91d are provided at the opposing locations of the hinge 92.

Next, a procedure for mounting the electrode 2 on the tube 8 by using the attachment device 9 is described.

First, as shown in Fig. 2, the electrode divided body 21 is fittingly inserted into an inside of the holding body 91 through the opening 91a.

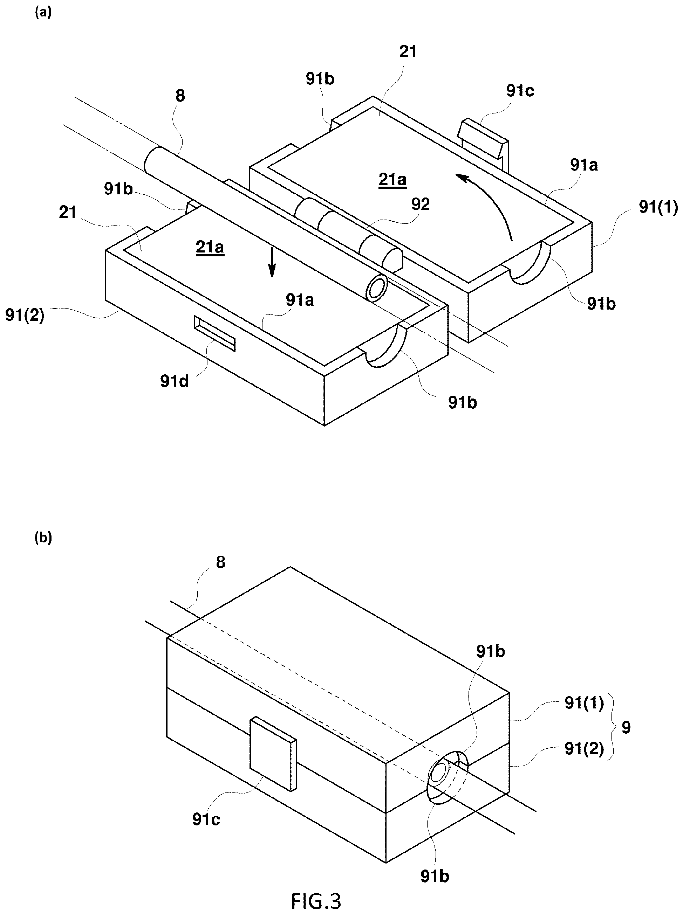

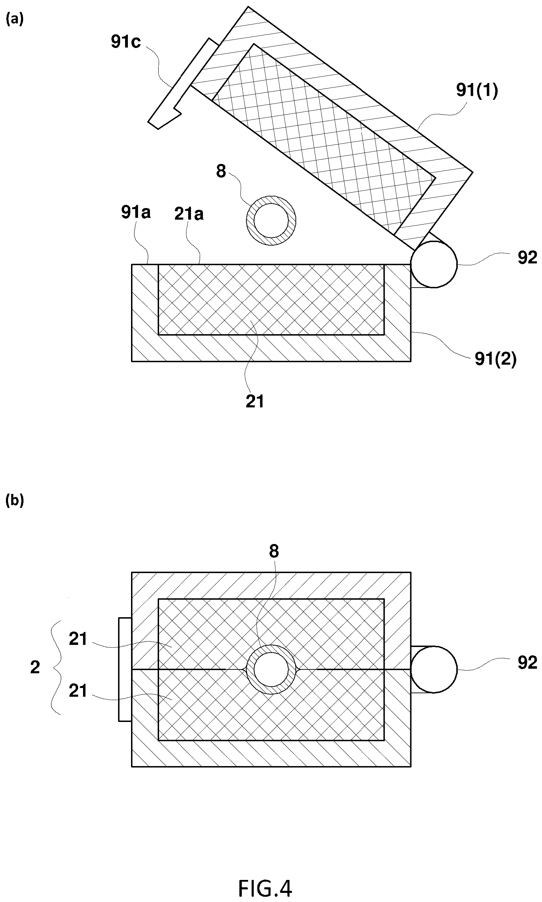

Next, the holding bodies 91 are positioned so as to clamp the tube 8, as shown in Fig. 3(a) and Fig. 4(a).

Then the holding bodies 91 are positioned gradually closer each other to clamp the tube 8. At this time, the tube 8 is positioned on the notches 91b.

When the holding bodies 91 are brought to approach further closer each other from this state, the electrode divided body 21 begins elastically deforming as it is pressed against a lateral surface of the tube 8, and the planar portion 21a begins to indent along the tube 8. The indented portion reduces a thickness of the electrode 2 (electrode divided body 21), namely a dimension along a direction of being clamped. Then, when the engaged state (mounted state) shown in Fig. 3(b) and Fig. 4(b) is reached, the electrode divided bodies 21 attach tightly to the entire circumference of the lateral surface of tube 8. In addition, the tube 8 clamped between the electrodes 2 extends outward through the notches 91b.

When the tube 8 is clamped in this manner, the tube 8 deforms in a direction that the tube 8 flattens viewed in cross-section. However, in the above-mentioned engaged state (mounted state), the elastic deformation or the thickness of the electrode 2 (electrode divided body 21) is determined so as to make the cross-sectional area of the tube 8 a predetermined percentage or more (50% or more, more preferably 70% or more, furthermore preferably 80% or more) compared to a natural state of the cross-sectional area. This is to avoid restricting blood flow in the blood circuit 12.

### 3. Voltage application section

The voltage application section 4 applies a high-frequency voltage between the arterial side electrode 2(1) and the venous side electrode 2(2). It can be configured, for example, using a function generator. In this embodiment, the voltage application section 4 is configured to apply a high-frequency voltage with a frequency of 50 kHz and a voltage of 10 Vp-p. However, the high-frequency voltage is not limited to this, and may be, for example, a high-frequency voltage with a frequency of 5 to 100 kHz and a voltage of 5 to 15 Vp-p.

### 4. Current measurement section

The current measurement section 5 converts the current flowing between the arterial side electrode 2(1) and the venous side electrode 2(2) to which the high-frequency voltage is applied into a voltage. The current measurement section 5 can be configured using a differential amplifier circuit that measures a potential difference across, for example, a 100 kΩ resistor 51. The resistance value of the resistor 51 is not limited to 100 kΩ.

### 5. Control processing section

### (1) Overview

A control arithmetic unit 6 comprises a CPU, an internal memory, input/output interfaces, an AD converter or the like. The control arithmetic unit 6 produces functions as a frequency component extraction section 61, a needle dislodgement detection section 62, and a reference value setting section 63 by the CPU and peripheral devices collaborating based on an inspection program stored in the internal memory. Additionally, the control arithmetic unit 6 controls the entire needle dislodgement detection device 100. The functions of this control arithmetic unit 6 may comprise a physically single computer or multiple physically separated computers.

### (2) Frequency component extraction section

The frequency component extraction section 61 extracts predetermined frequency components from the detection signal obtained by the current measurement section 5. The frequency component extraction section 61 may be configured, for example, using a lock-in amplifier or a bandpass filter. The frequency component extraction section 61 in this embodiment extracts a component having the same frequency as the high-frequency voltage applied by the voltage application section 4. Concretely, the frequency component extraction section 61 extracts the 50 kHz frequency component from the detection signal.

### (3) Needle dislodgement detection section

The needle dislodgement detection section 62 detects dislodgement of the arterial side puncture needle 7(1) or the venous side puncture needle 7(2) based on the extracted signal obtained by the frequency component extraction section 61. Concretely, the needle dislodgement detection section 62 detects dislodgement of the arterial side puncture needle 7(1) or the venous side puncture needle 7(2) by comparing the signal intensity indicated by the extracted signal obtained by the frequency component extraction section 61 with a predetermined reference value. The reference value in this embodiment is set by the reference value setting section 63.

As a method for detecting dislodgement by the needle dislodgement detection section 62 conceived are, for example: (1) in case that the signal intensity falls below the reference value; (2) in case that the signal intensity falls below a predetermined percentage (for example, 80%) of the reference value; or (3) in case that the signal intensity falls below the reference value for a predetermined number of times or for a predetermined duration continuously, as above-mentioned in (1) or (2).

### (4) Reference value setting section

The reference value setting section 63 sets the reference value using the extracted signal obtained by the frequency component extraction section 61 from the start of dialysis by the hemodialysis apparatus 10 until a predetermined time has passed. In this embodiment, the reference value setting section 63 sets the reference value immediately after the start of dialysis. The needle dislodgement detection by the needle dislodgement detection section 62 is not performed until the reference value is set by the reference value setting section 63. Then, after the predetermined time has passed (after the reference value has been set), the needle dislodgement detection section 62 detects dislodgement of the arterial side puncture needle 7(1) or the venous side puncture needle 7(2) by comparing the extracted signal obtained by the frequency component extraction section 61 with the reference value.

### Third Explanation of needle dislodgement detection method

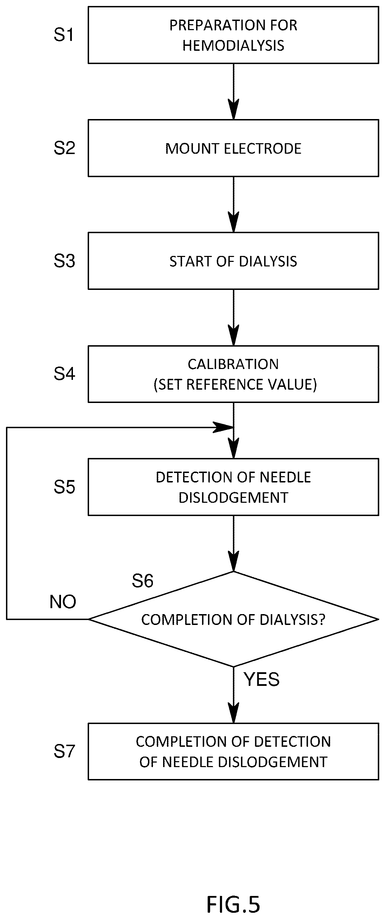

Next, the needle dislodgement detection method using the needle dislodgement detection device 100 of this embodiment will be described with reference to Fig. 5.

### 1. Mounting the electrodes

First, the arterial side puncture needle 7(1) and the venous side puncture needle 7(2) are punctured into the patient, completing preparation for hemodialysis (step S1). In this state, the electrodes 2 are mounted on each of the tubes 8 using the attachment device 9 (step S2). As mentioned above, regardless of the diameter of the tube 8, since the electrode divided body 21 deforms concavely, the electrode 2 is allowed to tightly attach to the outer circumferential surface of the tube 8.

### 2. Calibration (setting the reference value)

After the electrodes 2 are mounted on each of the tubes 8, a calibration operation is performed (step S4) simultaneously with the start of dialysis by the hemodialysis apparatus 10 (step S3). Concretely, the current measurement section 5 detects the potential difference between the electrode 2(1) and the electrode 2(2) by applying a high-frequency voltage to the arterial side electrode 2(1) and the venous side electrode 2(2) by the voltage application section 4. In addition, the frequency component extraction section 61 extracts the predetermined frequency component from the detection signal obtained by the current measurement section 5. Then, the reference value setting section 63 sets the reference value using the extracted signal obtained by the frequency component extraction section 61.

### 3. Detection of needle dislodgement

Simultaneously with the completion of the calibration in the above-mentioned (2), the needle dislodgement detection section 62 detects dislodgement of the arterial side puncture needle 7(1) or the venous side puncture needle 7(2) by comparing the extracted signal obtained by the frequency component extraction section 61 with the reference value set by the reference value setting section 63 (step S5). The detection of needle dislodgement continues until the hemodialysis by the hemodialysis apparatus 10 is completed (step S6, step S7). In the above-mentioned (2) and (3), the voltage application section 4 continuously applies the high-frequency voltage between the electrode 2(1) and the electrode 2(2) and the detection of the potential difference by the current measurement section 5 and the extraction of the frequency component by the frequency component extraction section 61 are performed on a constant basis.

### Forth Effect of this embodiment

In accordance with the needle dislodgement detection device 100 of this embodiment, even if the diameter of the tube 8 changes, since the electrode 2 itself deforms elastically to almost tightly attach to the outer circumferential surface of the tube 8, it is possible to mount the same electrode 2 in a tightly attached state on the tubes 8 of various different diameters.

Therefore, unlike conventional configuration, there is no need to prepare the electrodes 2 tailored to the diameter of the tube 8 so that the standardization of components is promoted. Furthermore, since the electrode 2 attaches tightly to the tube 8, it is possible to secure the same needle dislodgement detection accuracy as that of the conventional configuration.

In addition, since various noises superimposed on the current measurement signal such as a noise generated by vibration of the blood pump 14c of the hemodialysis apparatus 10 or a noise generated when a person touches the tube 8 can be effectively removed, it is possible to improve the accuracy of the needle dislodgement detection.

Furthermore, since the electrode 2 comprises two electrode divided bodies 21, and the electrode divided bodies 21 change a state from a non-engaged state where the electrode divided bodies 21 are separated each other to an engaged state where each of the divided bodies 21 clamps the tube 8 from the surrounding area of the tube 8, the electrode 2 can be easily mounted later on the tube 8 even after the puncture of the needle.

Although the electrode 2 deforms elastically, it is difficult to stably mount the electrode 2 on the tube 8. However, each of the electrode divided bodies 21 can be securely mounted on the tube 8 by clamping the tube 8 from the surrounding area of the tube 8 by the holding body 91 of high-rigidity.

### Fifth Modified embodiment

The present invention is not limited to the above-mentioned embodiments.

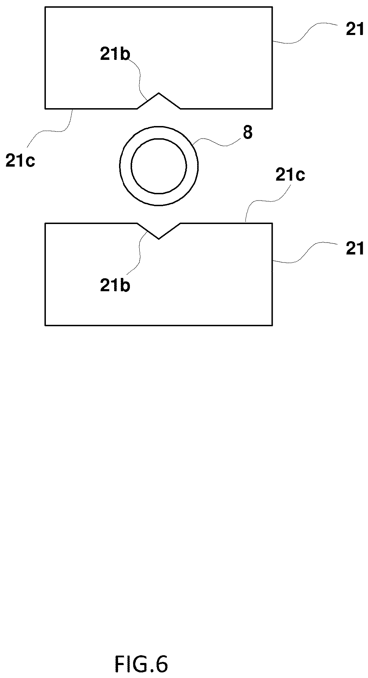

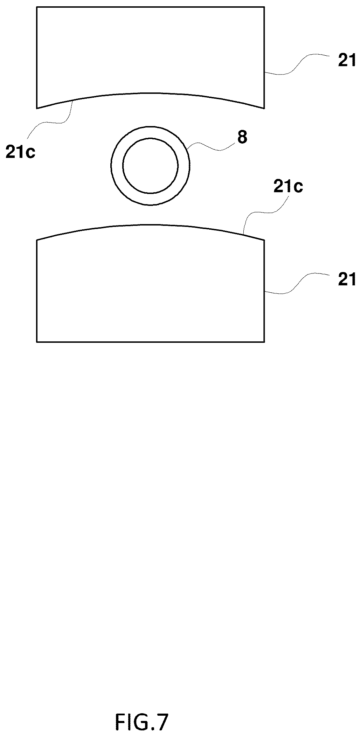

Facing surfaces 21c of the electrode divided bodies 21 need not be planar. For example, as shown in Fig. 6, a groove 21b may be provided on each of the facing surfaces 21c of the electrode divided bodies 21 that contact the tube 8. The cross-sectional shape of groove 21b is not limited to this shape. In addition, as shown in Fig. 7, the facing surfaces 21c of the electrode divided bodies 21 may be protruded or concave. The facing surfaces 21c of the electrode divided bodies 21 may also have mutually different convex and concave shapes. In short, the electrode divided body 21 may be configured such that, when clamping the tube 8, the electrode divided body 21 elastically deforms from its natural state so that each of the facing surfaces 21c contacts the tube 8 to become concave.

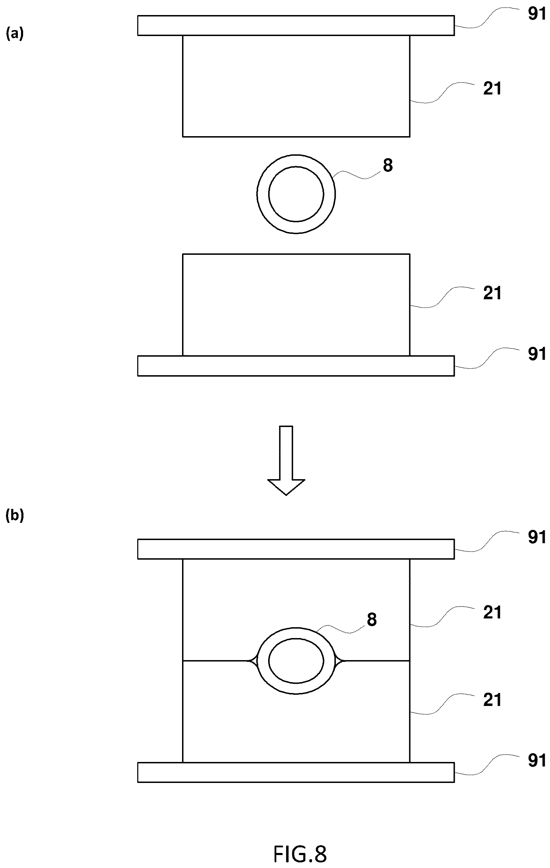

In addition, as shown in Fig. 8, the holding body 91 may be, for example, a flat plate adhered to a back surface of the electrode dividing body 21. As shown in Fig. 8, each of the holding bodies 91 need not be hinged and may be completely separable. In this case, each of the holding bodies 91 may be clamped together using a separate elastic connecting member such as a rubber band, or the above-mentioned engaging portions and engaged portions may be provided between the opposite sides of each of the holding bodies 91.

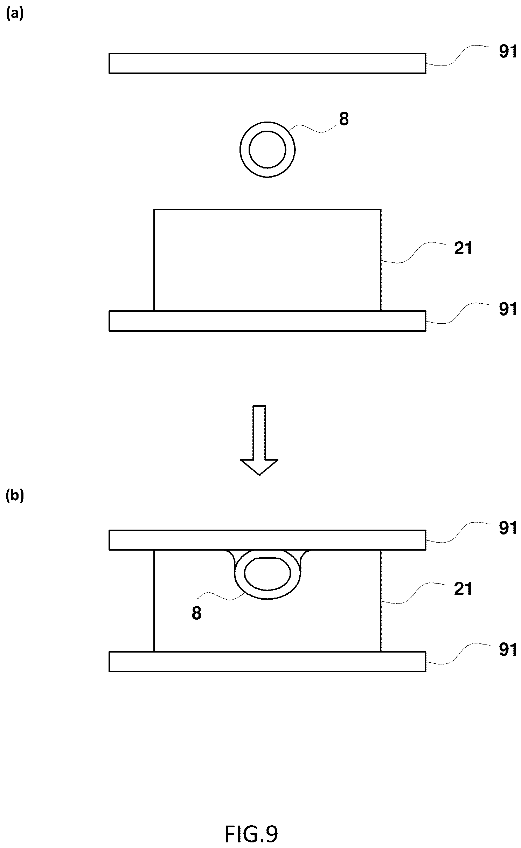

Furthermore, as shown in Fig. 9, the electrode 2 may not be divided and may be integrally formed. In this case, the tube 8 may be configured to be clamped between the holding body 91 and the electrode 2 from opposite directions.

Detection of needle dislodgement may be performed by detecting a simple change in the measurement current alone without performing the above-mentioned frequency component extraction.

It goes without saying that the present invention is not limited to the above-mentioned embodiments and various modifications are possible within the scope of the invention without departing from its spirit.

### [Explanation of codes]

- 100: needle dislodgement detection device
- 10: hemodialysis apparatus
- 2: electrode
- 21: electrode divided body
- 7: puncture needle
- 8: tube
- 9: attachment device
- 91: holding body

## Claims

1. A needle dislodgement detection device that detects dislodgement of an arterial side puncture needle or a venous side puncture needle of a hemodialysis apparatus from a patient, comprising
a pair of electrodes detachably mounted on an outer peripheral surface of an arterial side tube connected to the arterial side puncture needle and an outer peripheral surface of a venous side tube connected to the venous side puncture needle respectively,
a voltage application section that applies a predetermined voltage between the electrodes,
a current measurement section that measures a current flowing between the electrodes due to the applied voltage, and
a needle dislodgement detection section that detects dislodgement of the arterial side puncture needle or the venous side puncture needle based on a current measurement signal output by the current measurement section, and
**characterized by** that the electrodes are made of an elastically deformable conductive material and are mounted on the outer circumferential surfaces of the tubes by being pressed against the outer peripheral surfaces in a manner that accompanies elastic deformation.

2. The needle dislodgement detection device described in claim 1, wherein
the voltage application section applies a high-frequency voltage between the electrodes, and
the needle dislodgement detection section detects dislodgement of the arterial side puncture needle or the venous side puncture needle based on an extracted signal obtained by extracting a predetermined frequency component from the current measurement signal.

3. The needle dislodgement detection device described in claim 1, wherein
the electrode comprises a plurality of electrode divided bodies, wherein each of the electrode divided bodies is configured to clamp the tube from the outer circumferential surface of the tube in a mounted state that the electrode is mounted on the tube.

4. The needle dislodgement detection device described in claim 3, wherein
the electrode divided bodies are held by a plurality of holding bodies having higher rigidity than that of the electrode divided body, and the electrodes are configured to be in the mounted state by engaging the holding bodies with each other.

5. The needle dislodgement detection device described in claim 3, wherein
the electrode comprises a pair of the electrode divided bodies, and the electrode divided bodies clamp the tube from opposite directions in the mounted state.
